# EUROPEAN PATENT APPLICATION

(11) **EP 1 520 565 A2**
(43) Date of publication of application: **06.04.2005**
(21) Application number: 04256028.4
(22) Date of filing: 30.09.2004
(51) Int. Cl.: A61F 5/451, A61G 9/00

(54) **Device for disposing excrements**

(30) Priority: 30.09.2003 JP 2003340866; 01.12.2003 JP 2003401219
(71) Applicant: Aluvo Co., Ltd, Hamamatsu-shi, Shizuoka-ken 431-2103 (JP)
(72) Inventor: Kitamura, Teruo c/o ALUVO CO.,LTD., Hamamatsu-shi, Shizuoka-ken 431-2103 (JP)
(74) Representative: Waldren, Robin Michael

(57) **Abstract**

A device for disposing excrements of a person lying with the face upward, comprising: a cuplike body (1) having a rim provided with a sealing member (3) engageable with the body of the person to sealingly cover an anus region of the person such that a space for passing excrement of the person is defined between the cuplike body (1) and the person's body, a guide projection (2) extending obliquely downward in the direction opposite to the face of the lying person from the cuplike body (1) at a position facing the anus region of the lying person, the guide projection (2) being terminated in an opening; and a flexible tube (4) connected to the opening of the guide projection (2) for discharging the excrements therethrough.

## Description

This invention relates to a device for disposing excrements of a patient or a bedridden person.

Disposable diapers are conventionally used to collect excrements of bedridden persons or postoperative patients.

However, a wet and dirty diaper is uncomfortable to the user, and frequent changes of diapers impose a burden on caretakers. In addition, environmental pollution caused by incineration of wastes has become a serious problem in recent years and it is getting more difficult to discard a large amount of disposal diapers.

An excrement treating device having a diaper cup body, and a washing liquid feed hose and a suction hose which are connected to the diaper cup body is known (JP-A-H08-322868). Solid excrements in the diaper cup body are washed away with washing liquid and automatically sucked out through the suction hose. In this device, however, since a narrow open end of the suction hose is directly connected to a lower part of the diaper cup body and since solid excrements are discharged in a horizontal direction, the suction hose is easily clogged with the solid excrements. Also, since the hip of a user must be placed on a flat waist plate, the user cannot move and an aged person with bent back cannot use the device.

An excrement disposal device having a panty part, a pressing tube having two openings and attached to the panty part, and a discharge unit having a water and air supply port, a discharge port, a motor fan and a water detecting sensor (JP-A H06-54874) is also known. There is also proposed a wearing type disposable and washable toilet having a hip supporter having a discharge hole and provided with a polyvinyl bag for solid excrements, and a supporter having an abdomen supporter attached to a wearing belt and provided with a polyvinyl bag for liquid excrement attached to an end of a catheter (JP-A-H11-349). In these devices, however, since excrements are discharged in a horizontal direction, solid excrement is likely to cause clogging.

An excrement disposal device having a stool receiving cup and a urine receiving cup which can be separated from each other, discharge units connected to respective receiving cups to discharge excrements therefrom to the outside (JP-A-2003-153931) is also known.

The two receiving cups can be placed in optimum positions in accordance with the body shape of the user. However, since an end of the stool discharge tube is directly connected to a partition in the stool receiving cup and is the solid excrements are discharged in a horizontal direction, the hose is likely to be clogged.

The present invention has been made to solve the problems of the conventional excrement disposing devices.

In accordance with the present invention there is provided a device for disposing excrements of a person lying with the face upward, comprising:
a cuplike body having a rim provided with a sealing member engageable with the body of the person to sealingly cover an anus region of the person such that a space for passing excrements of the person is defined between said cuplike body and the person's body;
a guide projection extending obliquely downward in the direction opposite to the face of the lying person from said cuplike body at a position facing the anus region of the lying person, said guide projection being terminated in an opening; and
a flexible tube connected to said opening of said guide projection for discharging the excrements therethrough.

Since the guide projection extends obliquely downward from the cuplike body, the flexible tube connected to the guide projection is unlikely to be flattened. Also, since excrements of the person are allowed to fall down and smoothly guided through the guide projection and the flexible tube, the guide projection and the flexible tube are not easily clogged with the excrement.

The cuplike body and the sealing member may be configured such that the sealing member sealingly covers both anus and pubic regions of the person. In this case, since the pubic and anus regions of the person are covered with a single cuplike body, the device for disposing excrements can be easily attached to and removed from the body of the person.

The cuplike body and the guide projection may be integrated together and constructed to form an elbow pipe curved such that when the sealing member is in engagement with the person's body, the opening orients obliquely downward in the direction opposite to the face of the lying person. Since the elbow pipe is curved, the flexible tube connected to the elbow pipe is unlikely to be flattened. Also, since excrements of the person are allowed to fall down and smoothly guided through the guide projection and the flexible tube, the elbow pipe and the flexible tube are not easily clogged with excrements.

The present invention will be described in detail below with reference to the accompanying drawings, in which:
FIG. 1 is a perspective view, taken from below, illustrating a device for disposing excrements according to a first embodiment of this invention attached to the body of a person;
FIG. 2 is a perspective view, taken from above, illustrating the device for disposing excrements according to the first embodiment attached to the body of a person;
FIG. 3 is a side view illustrating the device for disposing excrements according to the first embodiment attached to the body of a person;
FIG. 4 is a cross-sectional view of a cuplike body of the first embodiment;
FIG. 5 is a perspective view of the cuplike body of the first embodiment;
FIG. 6 is a backside view of the cuplike body of the first embodiment;
FIG. 7 is a plan view of the cuplike body of the first embodiment;
FIG. 8 is a side view of the cuplike body of the first embodiment;
FIG. 9 is a fragmentary perspective view of an essential part of a sealing member of the first embodiment;
FIG. 10 is a perspective view of the sealing member of the first embodiment;
FIG. 11 is a backside view of the sealing member of the first embodiment;
FIG. 12 is a perspective view of an essential part of the device for disposing excrements according to the first embodiment;
FIG. 13 is a backside view of an essential part of the device for disposing excrements according to the first embodiment;
FIG. 14 is a side view of the device for disposing excrements according to the first embodiment;
FIG. 15 is a view of a flexible tube of the first embodiment;
FIG. 16 is a view of another example of the flexible tube;
FIG. 17 is a view of another example of the flexible tube;
FIG. 18 is a side view of a modified cuplike body;
FIG. 19 is a perspective view, taken from above, illustrating a device for disposing excrements according to a second embodiment of this invention attached to the body of a person;
FIG. 20 is a perspective view, taken from above, illustrating the device for disposing excrements according to the second embodiment attached to the body of a person;
FIG. 21 is a plan view of an elbow pipe of the second embodiment;
FIG. 22 is a perspective view of the elbow pipe of the second embodiment;
FIG. 23 is a side view of the elbow pipe of the second embodiment;
FIG. 24 is a cross-sectional view of the elbow pipe of the second embodiment;
FIG. 25 is a perspective view of essential parts of a cup and the elbow pipe of the second embodiment;

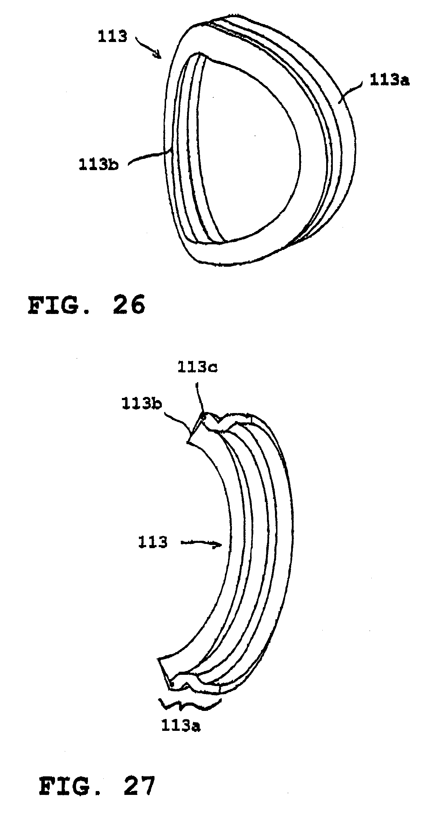

FIG. 26 is a perspective view of a sealing member of the second embodiment;
FIG. 27 is a perspective view, partially broken away, of the sealing member of the second embodiment; and
FIG. 28 is a perspective view illustrating the sealing member, the elbow pipe and a flexible tube of the second embodiment in an assembled state.

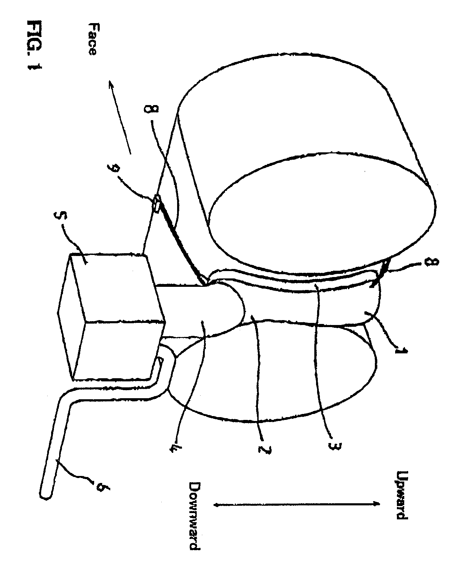

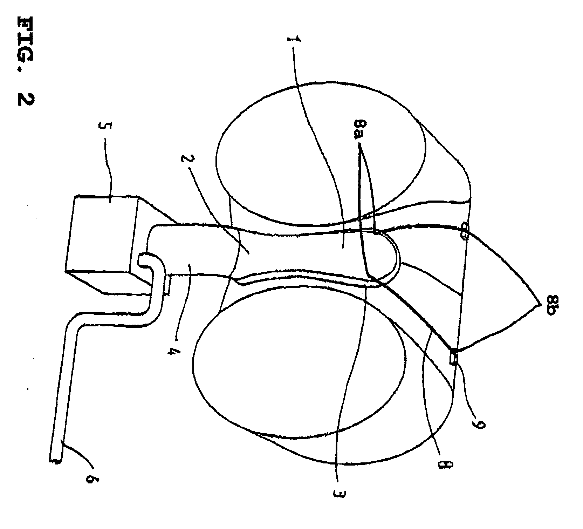

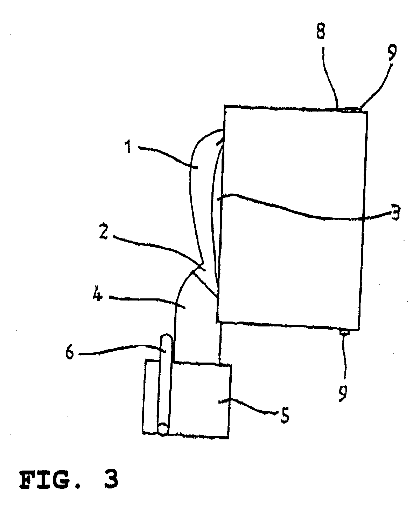

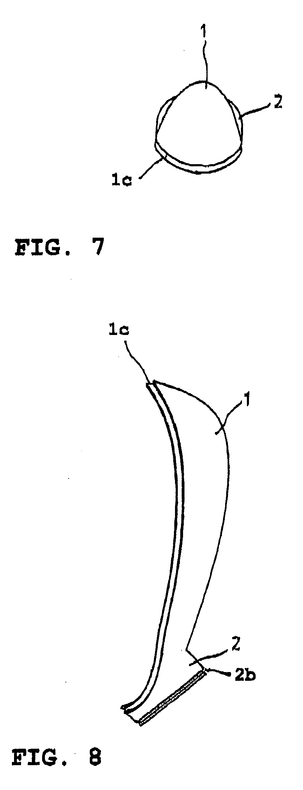

FIGS. 1 to 13 show a first embodiment of this invention.

As shown in FIGS. 1 to 4, a device for disposing excrements of a person lying with the face (not shown) upward according to a first embodiment of this invention has a cuplike body 1 having a rim 1a provided with a sealing member 3 engageable with the body of the person to sealingly cover the anus and pubic regions of the person such that a space for passing excrement of the person is defined between the cuplike body 1 and the person's body, a guide projection 2 extending obliquely downward in the direction opposite to the face of the lying person from the cuplike body 1 at a position facing the anus region of the lying person and terminated in an opening 2a, a flexible tube 4 connected to the opening 2a of the guide projection 2 for discharging the excrements therethrough. A connecting tube 6 is connected to the flexible tube 4 via an attachment part 5 within which a pulverizing device and a delivering pump (not shown) are housed.

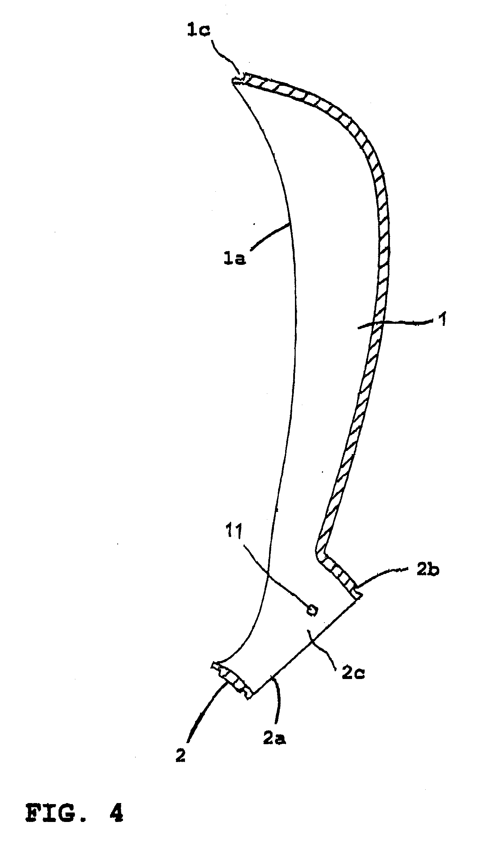

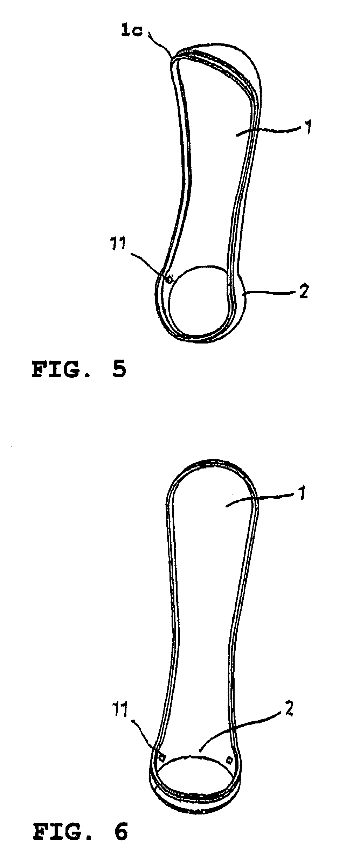

The cuplike body 1, which is made of an elastic material such as a soft rubber or soft plastic so as to be comfortable to wear, is elongated and curved convexly in the direction opposite to the face of the lying person as shown in FIGS. 4 to 8. An intermediate portion of the cuplike body 1, that is, the portion between the part for covering the pubic region of the person and the part for covering the anus region of the person is slightly narrower in width than the part for covering the pubic region of the person and the part for covering the anus region of the person (FIG. 6).

The cuplike body 1 has a thin wall portion 1c for attaching the sealing member 3 along the rim 1a (FIG. 4) .

The device of this embodiment has attaching means for attaching it to the body of the person. The attaching means may be any conventional means for attaching this kind of device to the body of a user such as a belt or supporter. The device of this embodiment preferably has connecting straps 8 and adhesive sheets 9 as the attaching means. As shown in FIGS 1 to 3, one ends 8a of the connecting straps 8 are secured to both sides of an upper part and a lower part of the cuplike body 1, respectively, and the adhesive sheets 9 are secured to the other ends 8b of the connecting straps 8.

Each of the adhesive sheets 9 is made of a flexible material and has a body contacting surface on which an adhesive material such as a polyurethane gel is applied. Since there is no need for a belt or the like which constricts the body of the person, the device is comfortable to wear.

The guide projection 2 is made of a material which is harder than the cuplike body 1 such as a hard rubber or hard plastic so that it cannot be easily deformed and get clogged. The guide projection 2 has an engaging groove 2b formed in a circumference along the opening 2a.

A multiplicity of sensors (not shown) for detecting passage of stool based on capacitance or a CCD image are provided in the wall of the guide projection 2.

A plurality of washing liquid supply pipes with injection ports 11 for injecting washing liquid therethrough are embedded in the wall of the guide projection 2. The injection ports 11 are formed in an inner surface 2c of the guide projection 2 at spaced positions (FIGS. 4 to 6).

The direction in which washing liquid is injected from the injection ports 11 is inclined with respect to the circumferential direction of the guide projection 2 and directed toward the opening 2a. when the sensors detect passage of stool, warm washing liquid or washing liquid containing a digestive enzyme which decomposes proteins or fats as needed is injected from the injection ports 11. The flows of washing liquid injected from the injection ports 11 flow spirally on the inner surface 2a along different routes to wash the body of the person covered with the cuplike body 1. The injection ports 11 may be positioned such that washing liquid flows in the axial direction of the guide projection 2 on the inner surface 2c of the guide projection 2.

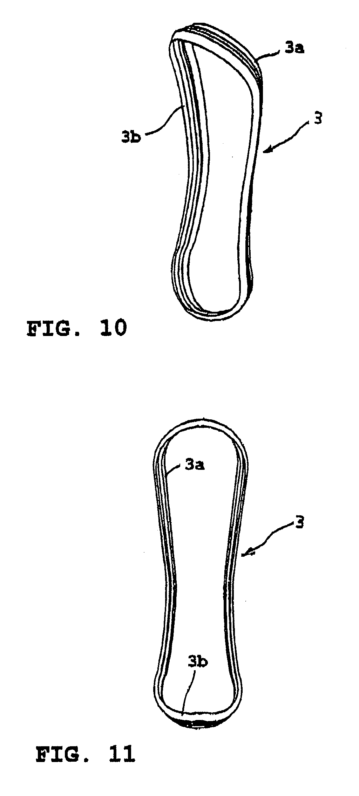

The sealing member 3 is preferably a ring-shaped member made of an elastic material such as a soft plastic and having a shape corresponding to the shape of the rim 1a of the cuplike body 1 as shown in FIGS. 10 and 11.

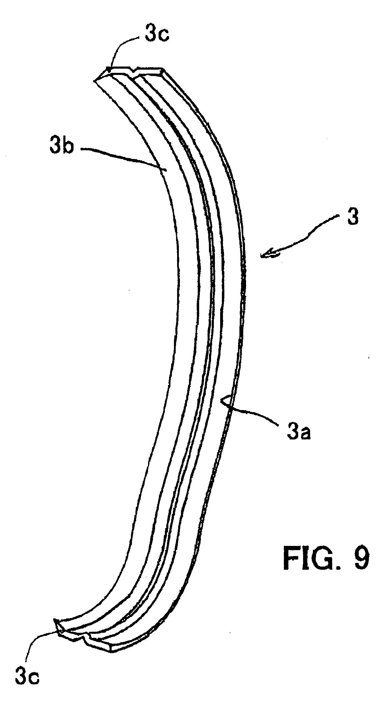

The sealing member 3 has a ring portion 3a extending from the rim 1a of the cuplike body 1, and a flange 3b extending radially inward from a peripheral end of the ring portion 3a. Preferably, the ring portion 3a is pleated and has a zigzag cross-section as shown in FIG. 9. When the ring portion 3a is pleated, the pressure applied to the sealing member 3 when the sealing member 3 is pressed against the body of the person can be uniformly dispersed over the flange 3b. Since the pleated ring portion 3a is folded flat by the pressure to attach the sealing member 3 to the body of the person, excrement is not caught in the pleated ring portion 3a.

The ring portion 3a of the sealing member 3 can be removably fitted over the thin wall portion 1c of the cuplike body 1. Thus, the sealing member 3 can be easily replaced when deteriorated.

The flange 3b of the sealing member 3 is tapered from base to edge. A flexible reinforcement 3c is embedded in a circumference of the flange 3b to enhance the strength of the flange 3b. When the flange 3b is brought into close contact with the body of the person and a high pressure is applied to the flange 3b, the reinforcement 3c is deformed and keeps the flange 3b in a shape which closely fits the body of the person to prevent fluid leakage between the flange 3b and the body of the person.

The reinforcement 3c may be a metal wire or a metal plate.

The flexible tube 4 is made of an elastic plastic material such as a soft foamed polyurethane, and has an inner surface 4a and an outer surface 4b. The inner surface 4a of the flexible tube 4 is smoothed by, for example, applying a coating to prevent adhesion of excrement thereto.

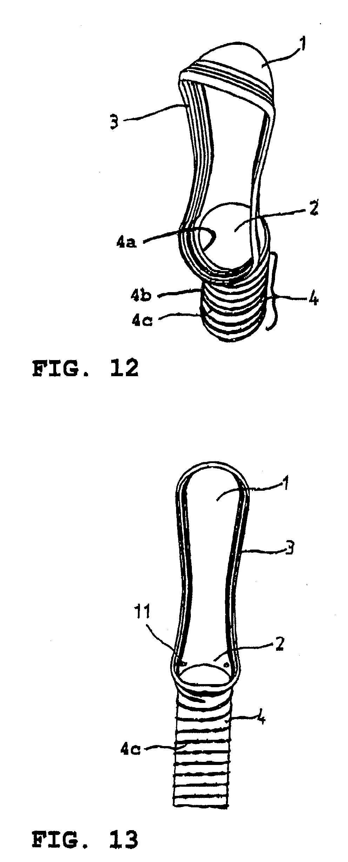

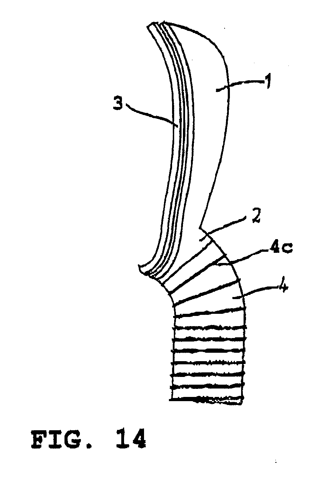

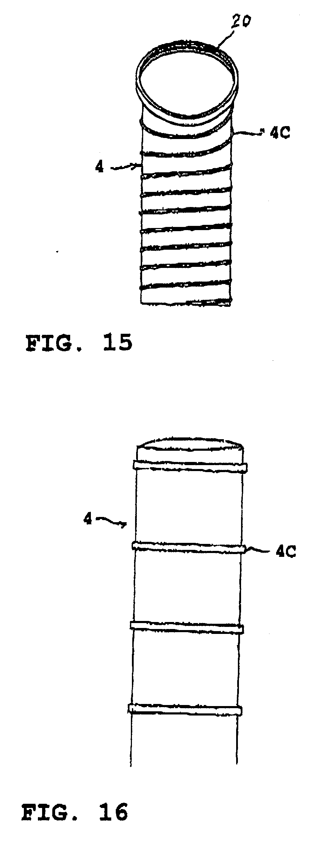

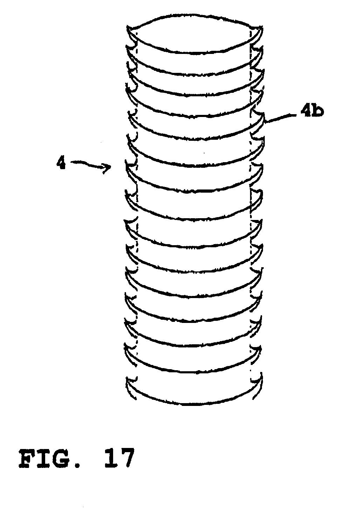

As shown in FIGS. 12 to 14, a reinforcing wire 4c is provided on the outer surface 4b or in the wall of the flexible tube 4 to enhance the strength of the flexible tube 4 so that the flexible tube 4 cannot be bent and get clogged. The reinforcing wire 4c may be provided in a spiral form on the outer surface 4b or in the wall of the flexible tube 4 (FIG. 15), or ring shaped reinforcing wires 14 may be provided at certain intervals (FIG. 16). Alternatively, projections and depressions may be formed on the outer surface 4b of the flexible tube 4 (FIG. 17).

One end of the flexible tube 4 is engaged with the engaging groove 2b of the guide projection 2, whereby the flexible tube 4 is rotatably connected to the guide projection 2. Since the guide projection 2 and the flexible tube 4 can be rotated relative to each other, the position of the flexible tube 4 can be changed depending on the body shape or posture of the person. Thus, the flexible tube can be prevented from being flattened and getting clogged.

The other end of the flexible tube 4 is rotatably connected to the attachment part 5 provided at the base of the connecting tube 6 as shown in FIGS. 1 to 3. Since the flexible tube 4 is made of an elastic material, it can be deformed depending on the position of the attachment part 5 and so on.

The connecting tube 6 is fixed on the floor or the like and connected to a sewer pipe or toilet.

A pulverizing device and a delivering pump are disposed in the attachment part 5. The pulverizing device has a cutter mechanism having piano wires crossed each other at the delivery port thereof so that excrement can be pulverized by the delivery pressure of the pulverizing pump and the cutter mechanism.

To attach the device to the body of a person, the sealing member 3 is once removed from the cuplike body 1. Then, the flange 3b of the sealing member 3 is positioned around the pubic and anus regions of the person and the cuplike body 1 is connected to the sealing member 2. The flexible tube 4 has been connected to the cuplike body 1. Since the sealing member 3 can be removed from the cuplike body 1, the sealing member 3 can be placed in the optimum position on the body of the person easily and, consequently, the device can be placed in the optimum position easily.

Then, the adhesive sheets 9 are stuck to the body of the person and the connection straps 8 are stretched with an appropriate tension to bring the sealing member 3 into pressure contact with the body of the person.

When there is a gap between the flange 3b of the sealing member 3 and the body of the person, a pressure is applied with a hand to deform the reinforcement 3c so that the flange 13 of the sealing member 3 can fit the body of the person.

When the person evacuates excrement, the sensors provided in the guide projection 2 detect the excrement. Then, washing liquid is injected from the injection ports 11 to wash away the excrement. Since the excrement is washed away, adhesion of filth or generation of odor can be prevented.

Also, since the guide projection 2 extends obliquely downward from the cuplike body 1, the excrement is guided into the flexible tube 4 smoothly. Then, the excrement is pulverized by the pulverizing device and the delivering pump in the attachment part 5 and discharged to a sewer pipe or the like through the connecting tube 6.

The cuplike body 1 may be provided with a nozzle for injecting warm water to the pubic and anus regions of the person.

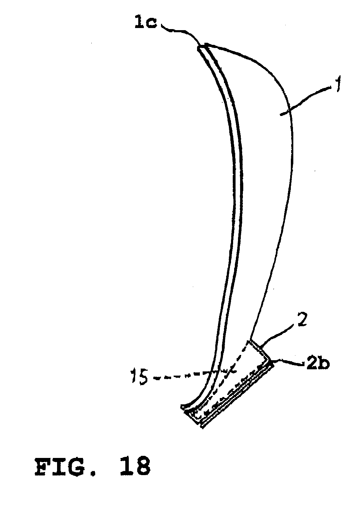

The above first embodiment may be modified in various manners. For example, the guide projection 2 may be made of an elastic material integrally with the cuplike body 1. In this case, as shown in FIG. 18, a hollow tube 15 of a hard material such as a hard plastic is provided in the guide projection 2 to prevent the guide projection from being fluxed. The hollow tube 15 may be embedded in the wall of the guide projection 2 or laminated on the inner surface of the guide projection 2. The cuplike body 1 and its associated sealing member 3 may be configured such that the sealing member 3 sealingly covers only the anus region of the person.

Description will be made of a second embodiment of this invention, in which the same reference numerals as those in the first embodiment designate similar component parts and their description will be omitted.

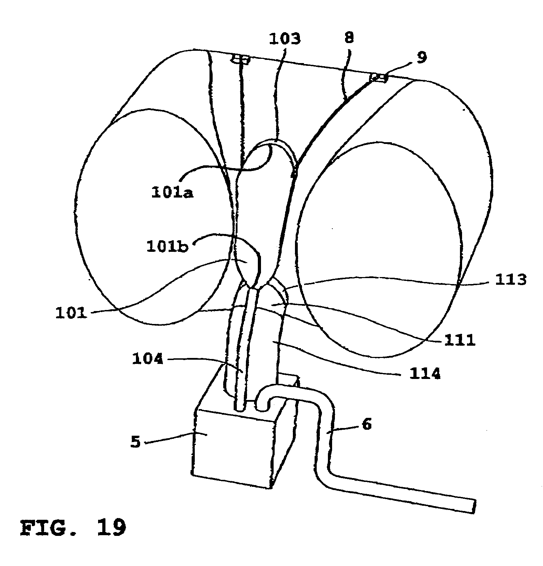

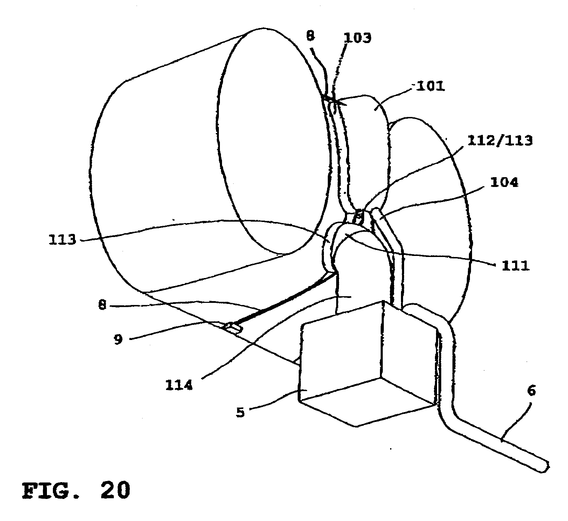

As shown in FIGS. 19 and 20, a device according to the second embodiment of this invention has a cup 101 having a rim 101a provided with a sealing member 103 engageable with the body of a person to sealingly cover the pubic region of the person and terminated in an opening 101b, and a flexible pipe 104 connected to the opening 101b of the cup 101 for discharging urine of the person therethrough. The device also has an elbow pipe 111 having first and second openings 111a and 111b, a sealing member 113 secured to the first opening (or rim) 111a of the elbow pipe 111 and engageable with the body of the person to sealingly cover the anus region of the person, and a flexible tube 114 connected to the second opening 111b of the elbow pipe 111 for discharging excrements of the person therethrough. A connecting tube 6 is connected to the flexible pipe 104 and the flexible tube 114 via an attachment part 5 within which a pulverizing device and a delivering pump (not shown) are housed.

The cup 101 and the elbow pipe 111 are made of a hard material such as a hard plastic.

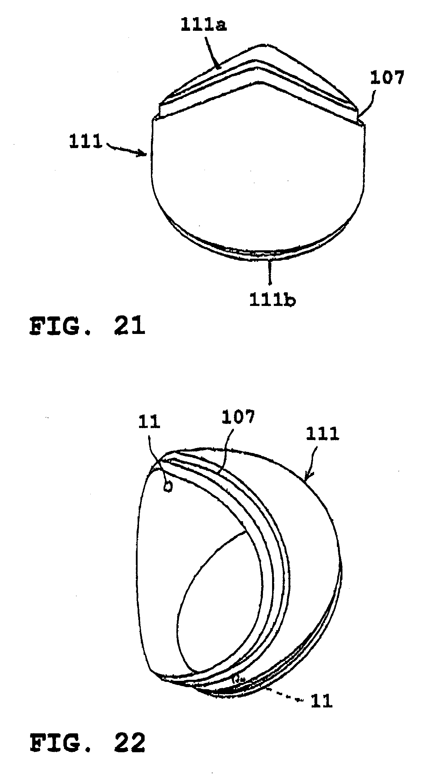

The rim 101a of the cup 101 has a more elongated shape than the first opening 111a of the elbow pipe 111 does so that it can fit the pubic region of the person. As shown in FIGS. 21 and 22, the lateral center portion of the first opening 111a of the elbow pipe 111 is shaped so that they can fit the groin area of the person.

Since the cup 101 and the elbow pipe 111 are separated, the device can cope with the variations of the body shape of users.

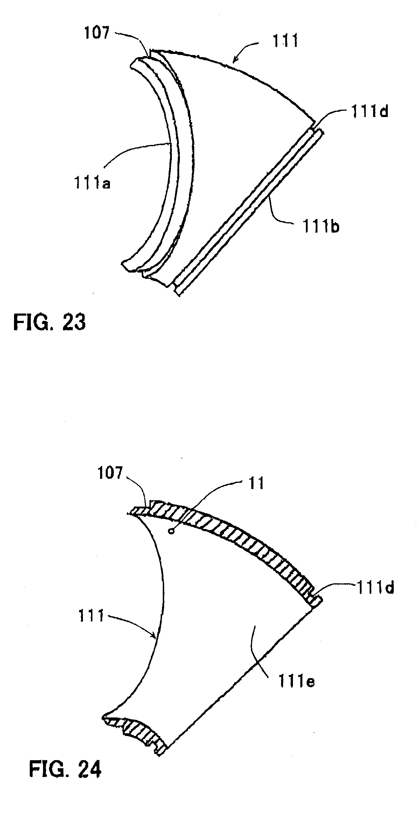

The elbow pipe 111 has a thin wall portion 107 for attaching the sealing member 113 along the first opening 111a and an engaging groove 111d for engaging with an end of the flexible tube 114 in a circumference along the second opening 111b as shown in FIGS. 23 and 24. The cup 101 also has a thin wall portion for attaching the sealing member 103 along the rim 101a and an engaging groove for engaging with one end of the flexible pipe 104 in a circumference along the opening 101b although not shown since the cup 101 and the elbow pipe 111 are generally the same in structure except for the dimensions.

A multiplicity of sensors (not shown) for detecting passage of excrements based on capacitance or a CCD image are provided in the wall of the elbow pipe 111.

A plurality of washing liquid supply pipes with water injection ports 11 for injecting washing liquid therethrough are embedded in the wall of the elbow pipe 111. The water injection ports 11 are formed in an inner surface 111e of the elbow pipe 111 at spaced positions (FIG. 24).

The direction in which water is injected from the water injection ports 11 is inclined with respect to the circumferential direction of the elbow pipe 111 toward the second opening 111b. When the sensors detect passage of excrements, warm washing liquid or washing liquid containing a digestive enzyme which decomposes proteins or fats as needed is injected from the water injection ports 11. Water flows injected from the water injection ports 11 flow spirally on the inner surface 111e of the elbow pipe 111 along different routes.

The water injection ports 11 may be positioned such that washing liquid flows in the axial direction of the elbow pipe 111 on the inner surface 111e of the elbow pipe 111.

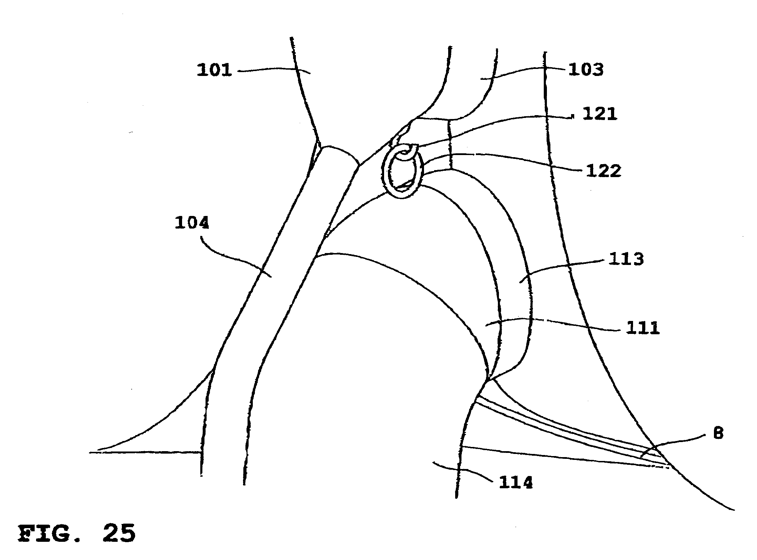

As shown in FIG. 25, a pair of connectors 121 and 122, which are removably engageable with each other, are attached to the opposed parts of the cup 101 and the elbow pipe 111, respectively, so that the cup 101 and the elbow pipe 111 can be removably connected to each other. Since the cup 101 and the elbow pipe 111 can be separated when attached to or removed from the body of the person and stable connected while in use, the device is easy to handle.

The device of this embodiment has connecting straps 8 and adhesive sheets 9 as attaching means for attaching it to the body of the person. One ends 8a of the connecting straps 8 are secured to both sides of an upper part of the cup 101 and a lower part of the elbow pipe 111, respectively, and adhesive sheets 9 are secured to the other ends 8b of the connecting straps 8.

The sealing members 103 and 113 are generally the same in structure as the sealing member 3 in the first embodiment. That is, the sealing member 103 has a ring portion, a flange and a reinforcement embedded in a circumference thereof, although not shown. Similarly, the sealing member 113 has a ring portion 113a, a flange 113b and a reinforcement 113c embedded in a circumference thereof.

The flexible pipe 104 and the flexible tube 114 are generally the same in structure as the flexible tube 4 in the first embodiment. That is, the flexible pipe 104 has a smoothed inner surface 104a, an outer surface 104b and a reinforcing wire provided on the outer surface 104b or in the wall thereof and rotatably connected to the cup 101, and the flexible tube 114 has a smoothed inner surface 114a, an outer surface 114b and a reinforcing wire 114c provided on the outer surface 114b or in the wall thereof and rotatably connected to the elbow pipe 111.

To attach the device for disposing excrements to the body of a person, the sealing members 103 and 113 are once removed from the cup 101 and the elbow pipe 111. Then, the flange of the sealing member 103 is positioned around the pubic region of the person and the cup 101 is connected to the sealing member 103.

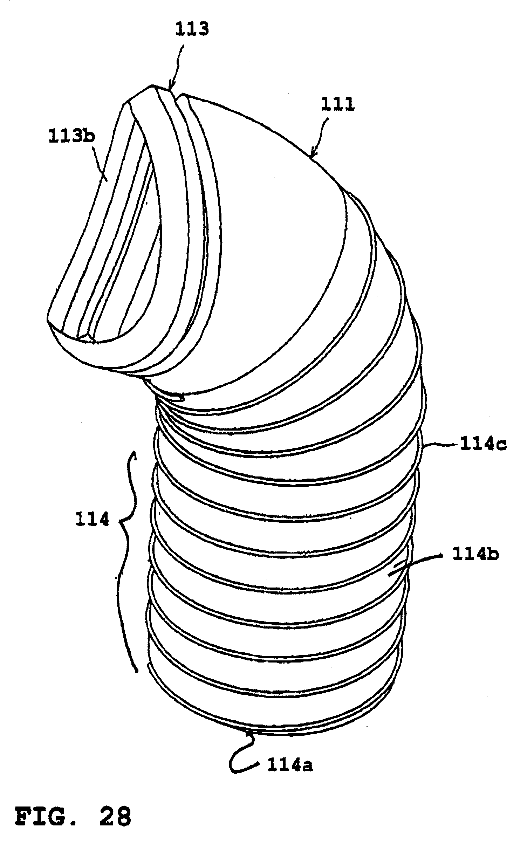

Also, the flange 113b of the sealing member 113 is positioned around the anus region of the person and the elbow pipe 111 is connected to the sealing member 113 (FIG. 28). The flexible pipe 104 and the flexible tube 114 have been connected to the cup 101 and the elbow pipe 111, respectively.

Then, the adhesive sheets 9 are attached to the body of the person and the connection straps 8 are stretched with an appropriate tension to bring the sealing members 103 and 113 into pressure contact with the body of the person. The paired connector 121 and 122 are connected to connect the cup 101 and the elbow pipe 111.

When there is a gap between the flanges and the body of the person, a pressure is applied with a hand to deform the reinforcement so that the flanges of the sealing members 103 and 113 can fit the body of the person.

When the person evacuates stool, the sensors provided in the elbow pipe 111 detects the stool. Then, washing liquid is injected from the water injection ports 11 to wash away the stool.

Since the elbow pipe 111 is curved in the shape of an elbow, the excrements are guided into the flexible tube 114 smoothly and discharged to a sewer pipe or the like through the connecting tube 6.

The cup 101 and the elbow pipe 111 may have nozzles for injecting warm water to the pubic region and the anus region of the person.

The paired connectors provided on the cup 101 and the elbow pipe 111 can be engaged at a plurality of positions so that the cup 101 and the elbow pipe 111 can be easily attached and removed and the distance between them can be adjusted.

The second embodiment may be modified in various manners as in the first embodiment. Further, the cup 101 and its associated parts for disposing urine may be removed and only the elbow pipe 111 and its associated parts for disposing solid excrements may be used by themselves. Such a modified embodiment of the second embodiment may be regarded as being similar to a modified embodiment of the above-described first embodiment in which the cuplike body 1 and its associated sealing member 3 are configured such that the sealing member 3 sealingly covers only the anus region of the person.

In the foregoing embodiments, since the guide projection 2 and the elbow pipe 111 extend obliquely downward toward the feet of the lying person, the excrements are easily guided downward by gravity and are easily discharged through the flexible tube 4 or 114 which generally extends downward. Thus, although not shown, the bed on which the person lies has desirably a bore or a through hole to provide a space for the receipt or passage of the flexible tube 4 and the guide projection 2 or the elbow pipe 111.

## Claims

1. A device for disposing excrements of a person lying with the face upward, comprising:
a cuplike body having a rim provided with a sealing member engageable with the body of the person to sealingly cover an anus region of the person such that a space for passing excrements of the person is defined between said cuplike body and the person's body;
a guide projection extending obliquely downward in the direction opposite to the face of the lying person from said cuplike body at a position facing the anus region of the lying person, said guide projection being terminated in an opening; and
a flexible tube connected to said opening of said guide projection for discharging the excrements therethrough.

2. A device as claimed in claim 1, wherein said cuplike body and said sealing member are configured so that said sealing member sealingly covers both anus region and pubic regions of the person.

3. A device as claimed in claim 1 or 2, wherein said flexible tube is rotatably connected to said opening of said guide projection.

4. A device as claimed in claim 1 or 2, wherein said cuplike body is made of an elastic material and said guide projection is made of a hard material.

5. A device as claimed in claim 1 or 2, wherein each of said cuplike body and said guide projection is made of an elastic material and a hollow tube of a hard material is provided in said guide projection to prevent said guide projection from being fluxed.

6. A device as claimed in claim 1 or 2, wherein said sealing member comprises an elastic ring portion extending from said rim of said cuplike body, and an elastic flange extending radially inward from a peripheral end of said ring portion and tapered from base to edge.

7. A device as claimed in claim 1 or 2, wherein said flange of said sealing member has a flexible reinforcement embedded in a circumference thereof for keeping said flange in a desired deformed state.

8. A device as claimed in claim 1 or 2, wherein said guide projection has an inner surface having a plurality of injection ports for injecting washing liquid therethrough to wash away the excrements.

9. A device as claimed in claim 1 or 2, wherein said flexible tube has a smoothed inner surface and a reinforcing wire provided on an outer surface or in the wall thereof.

10. A device as claimed in claim 1 or 2, further comprising connecting straps having one ends connected to the cuplike body, and adhesive sheets which are secured to the other ends of said connecting straps and can be stuck to the body of the person.

11. A device as claimed in claim 1, wherein said cuplike body and said guide projection are constructed to form an elbow pipe curved so that when said sealing member is in engagement with the person's body, said opening orients obliquely downward in the direction opposite to the face of the lying person.

12. A device as claimed in claim 11, wherein said flexible tube is rotatably connected to said opening of said elbow pipe.

13. A device as claimed in claim 11, wherein said sealing member for said elbow pipe comprises an elastic ring portion extending from said rim of said elbow pipe and an elastic flange extending radially inward from a peripheral end of said ring portion and tapered from base to edge.

14. A device as claimed in claim 11, wherein said flange of said sealing member for said elbow pipe has a flexible reinforcement embedded in a circumference thereof for keeping said flange in a desired deformed state.

15. A device as claimed in claim 11, wherein said elbow pipe has an inner surface having a plurality of injection ports for injecting washing liquid therethrough to wash away the excrements.

16. A device as claimed in claim 11, wherein said flexible tube has a smoothed inner surface and a reinforcing wire provided on an outer surface or in the wall thereof.

17. A device as claimed in claim 11, further comprising connecting straps having one ends connected to said elbow pipe, and adhesive sheets which are secured to the other ends of the connecting straps and can be stuck to the body of the person.

18. A device as claimed in claim 11, further comprising a cup having a rim provided with a sealing member engageable with the body of a person to sealingly cover a pubic region of the person and terminated in an opening; and
a flexible pipe connected to said opening of said cup for discharging urine of the person therethrough.

19. A device as claimed in claim 18, wherein said flexible pipe is rotatably connected to said opening of said cuplike body.

20. A device as claimed in claim 18, wherein said sealing member for said cup has an elastic ring portion extending from said rim of said cup and an elastic flange extending radially inward from a peripheral end of said ring portion and tapered from base to edge.

21. A device as claimed in claim 18, wherein said flange of said sealing member for said cup has a flexible reinforcement embedded in a circumference thereof for keeping said flange in a desired deformed state.

22. A device as claimed in claim 18, wherein said flexible pipe has a smoothed inner surface and a reinforcing wire provided on an outer surface or in the wall thereof.

23. A device as claimed in claim 18, further comprising connecting straps having one ends connected to said cup, and adhesive sheets which are secured to the other ends of the connecting straps and can be stuck to the body of the person.

24. A device as claimed in claim 18, further comprising a connector for removably connecting said elbow pipe and said cup.
